(19) Europäisches Patentamt
European Patent Office
Office européen des brevets

(11) Veröffentlichungsnummer: **0 080 080**
**A1**

(12) # EUROPÄISCHE PATENTANMELDUNG

(21) Anmeldenummer: **82110076.5**

(22) Anmeldetag: **02.11.82**

(51) Int. Cl.³: **C 07 D 413/12**, A 01 N 43/80

(30) Priorität: **12.11.81 DE 3144951**

(43) Veröffentlichungstag der Anmeldung: **01.06.83**
**Patentblatt 83/22**

(84) Benannte Vertragsstaaten: **AT BE CH DE FR GB IT LI NL SE**

(71) Anmelder: **BASF Aktiengesellschaft,**
**Carl-Bosch-Strasse 38, D-6700 Ludwigshafen (DE)**

(72) Erfinder: **Graf, Hermann, Dr., Ginsterstrasse 15, D-6704 Mutterstadt (DE)**
Erfinder: **Theobald, Hans, Dr., Parkstrasse 2, D-6703 Limburgerhof (DE)**
Erfinder: **Rentzea, Costin, Dr., Neuenheimer Landstrasse 72, D-6900 Heidelberg (DE)**
Erfinder: **Zeeh, Bernd, Dr., Thorwaldsenstrasse 5, D-6700 Ludwigshafen (DE)**
Erfinder: **Pommer, Ernst-Heinrich, Dr., Berliner Platz 7, D-6703 Limburgerhof (DE)**
Erfinder: **Ammermann, Eberhard, Dr., Sachsenstrasse 3, D-6700 Ludwigshafen (DE)**

(54) **Isoxazol-carbonsäure-amide, ihre Herstellung und ihre Verwendung als Fungizide.**

(57) Die Erfindung betrifft Isoxazolyl-carbonsäure-amide der Formel I

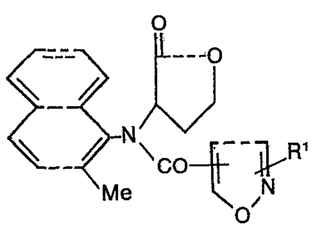

I,

in der R¹ für Wasserstoff, Methyl, Ethyl oder Chlormethyl steht, deren Herstellung und diese enthaltende Fungizide.

EP 0 080 080 A1

# Isoxazol-carbonsäure-amide, ihre Herstellung und ihre Verwendung als Fungizide

Die vorliegende Erfindung betrifft neue Isoxazolcarbonsäure-amide, Verfahren zur Herstellung, Fungizide, die diese Verbindungen als Wirkstoffe enthalten, sowie Verfahren zur Bekämpfung von Pilzen.

Heterocyclische Carbonsäureanilide, die fungizid wirksam sind, sind aus der DE-OS 25 13 732 und der DE-OS 25 13 788 bekannt. Heterocyclische Reste in diesen Verbindungen sind Pyridyl, Pyrimidinyl, Dihydro-pyranyl, Dihydro-1,4-oxathiinyl, Thienyl und Furyl. Die Wirkung dieser Verbindungen gegen Pilze der Ordnung Peronosporales ist jedoch unzureichend.

Es wurde nun gefunden, daß Isoxazolyl-carbonsäure-amide der Formel I

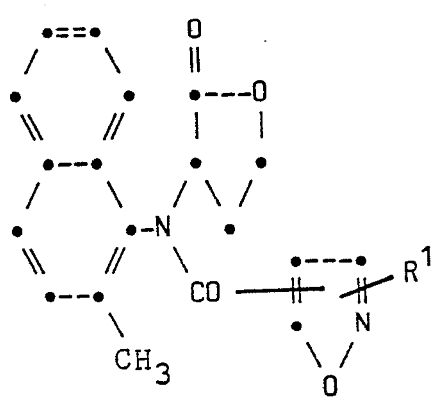

I,

in der $R^1$ für Wasserstoff, Methyl, Ethyl oder Chlormethyl steht, fungizid ausgezeichnet wirksam sind und bekannten heterocyclischen Carbonsäure-amiden in ihrer Wirkung, insbesondere gegen Pilze der Ordnung Peronosporales, überlegen sind.

Sws/P

BAD ORIGINAL

Die Isoxazol-carbonsäure-amide der Formel I besitzen ein Asymmetriezentrum im alpha-C-Atom des Butyrolacton-Teils. Zudem können Atropisomere durch eingeschränkte Drehbarkeit um die C-N-Bindung im Naphthylamin-Rest entstehen. Die optisch reinen Enantiomeren bzw. Diastereomeren können nach üblichen Methoden erhalten werden. Fungizid wirksam sind sowohl die bei der Synthese üblicherweise anfallenden Gemische als auch die reinen Enantiomeren.

Die Isoxazolyl-carbonsäure-amide der Formel I können durch Umsetzen von N-(alpha-Butyro-gamma-lactonyl)-2-methyl-1--naphthylamin mit einem Isoxazol-carbonsäure-derivat der Formel II

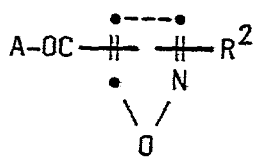

II,

in der $R^2$ für Wasserstoff, Methyl, Ethyl oder Chlormethyl und A für eine nucleophil verdrängbare Abgangsgruppe steht, erhalten werden.

In Formel II bedeutet A vorzugsweise Halogen, insbesondere Chlor oder Brom, Alkoxycarbonyloxyreste, insbesondere Methoxycarbonyloxy und Ethoxycarbonyloxy, oder den Benzyloxycarbonyloxyrest oder einen Azolylrest - insbesondere den Imidazolyl- oder den Triazolylrest.

Die Umsetzung kann in Gegenwart eines Lösungs- oder Verdünnungsmittels durchgeführt werden. Zu den bevorzugten Lösungs- bzw. Verdünnungsmitteln gehören Halogenkohlen-

BAD ORIGINAL

wasserstoffe, beispielsweise Methylenchlorid, Chloroform, 1,2-Dichlorethan, Chlorbenzol, aliphatische oder aromatische Kohlenwasserstoffe, wie Cyclohexan, Petrolether, Benzol, Toluol oder Xylole; Ester wie Essigsäureethylester; Nitrile, wie Acetonitril; Sulfoxide, wie Dimethylsulfoxid; Ketone, wie Aceton oder Methylethylketon; Ether, wie Diethylether, Tetrahydrofuran oder Dioxan oder Gemische dieser Lösungsmittel. Zweckmäßig verwendet man das Lösungs- bzw. Verdünnungsmittel in einer Menge von 100 bis 2000 Gew.%, vorzugsweise 100 bis 1000 Gew.%, bezogen auf die Ausgangsstoffe der Formel II bzw. III.

Es kann zweckmäßig sein, die Reaktion in Gegenwart von Basen durchzuführen. Geeignete anorganische oder organische Basen, die gegebenenfalls als säurebindende Mittel dem Reaktionsgemisch zugesetzt werden können, sind beispielsweise Alkalicarbonate, wie Kalium- oder Natriumcarbonat; Alkalihydride, wie Natriumhydrid oder tertiäre Amine, wie Trimethylamin, Triethylamin, N,N-Dimethylanilin, N,N-Dimethylcyclohexylamin, N-Methylpiperidin oder Pyridin; Azole, wie 1,2,4-Triazol oder Imidazol. Es können aber auch andere übliche Basen verwendet werden.

Die Umsetzung kann mit Reaktionsbeschleunigern beschleunigt werden. Als solche kommen vorzugsweise Metallhalogenide, wie Natriumbromid oder Kaliumiodid, Azole, wie Imidazol oder 1,2,4-Triazol oder Pyridine, wie 4-Dimethylaminopyridin oder Mischungen dieser Substanzen in Betracht. Zweckmäßigerweise setzt man auf 1 Mol Naphthylaminderivat 0,9 bis 1,3 Mol Säurederivat der Formel II sowie gegebenenfalls 0,5 bis 2 Mol Base und gegebenenfalls 0,01 Mol bis 0,1 Mol Reaktionsbeschleuniger ein.

Die Umsetzung wird im allgemeinen bei einer Temperatur im Bereich zwischen 0 und 120°C in einem Zeitraum von 1 bis

BAD ORIGINAL

60 h durchgeführt, drucklos oder unter Druck, kontinuierlich oder diskontinuierlich.

In einer bevorzugten Form des erfindungsgemäßen Verfahrens vermischt man N-(alpha-Butyro-gamma-lactonyl)-2-methyl-1-naphthylamin (N) gegebenenfalls mit einer Base und gegebenenfalls mit einem Verdünnungsmittel, gibt dann das Säurederivat der Formel II und gegebenenfalls den Reaktionsbeschleuniger zu und hält das Reaktionsgemisch für 0,5 bis 12, vorzugsweise 1 bis 6 h bei der Reaktionstemperatur, die zwischen 0 und 120°C liegen kann.

Zur Isolierung der neuen Verbindungen wird, falls vorhanden, das Verdünnungsmittel entfernt, der Rückstand wird dann in einem geeigneten Lösungsmittel gelöst und mit verdünnter Säure, danach mit wäßriger verdünnger Lauge sowie mit Wasser gewaschen, um die überschüssige Base und die Ausgangsstoffe zu entfernen.

Die nach dem Abdestillieren des Lösungsmittels verbleibenden Produkte bedürfen im allgemeinen keiner weiteren Reinigung, können aber, falls erforderlich, nach bekannten Methoden, beispielsweise durch Umkristallisation, Extraktion oder Chromatographie, weiter gereinigt werden.

Das Naphthylaminderivat sowie Verfahren zu seiner Herstellung sind aus der DE-OS 30 06 154 bekannt.

Die als Ausgangsstoffe eingesetzten Isoxazol-carbonsäurederivate der Formel II sind teilweise bekannt bzw. können nach bekannten Methoden aus den entsprechenden Isoxazol-carbonsäuren (Formel II, A = OH) hergestellt werden. Diese Isoxazol-carbonsäuren sind teilweise bekannt (Gazz. chim. ital. 72, 458-474 (1942); ibid. 73, 764-768 (1948)) oder können durch Oxidation der bekannten Carbinole

BAD ORIGINAL

(Gazz. chim. ital. 69, 536-539 (1939)), wie in DE-OS 29 40 189 beschrieben, hergestellt werden.

Das folgende Beispiel erläutert die Herstellung der Isoxazolyl-carbonsäure-amide der Formel I.

Beispiel 1

N-(alpha-Butyro-gamma-lactonyl)-N-(2-methyl-1-naphthyl)-isoxazol-5-carbonsäure-amid

19,2 g (80 mmol) N-(alpha-Butyro-gamma-lactonyl)-2-methyl-1-naphthylamin und 10,5 g (80 mmol) Isoxazol-5-carbonsäurechlorid reagierten in 100 ml trockenem Toluol 3 h bei 60°C, wobei ein Strom gereinigten Stickstoffs eingeleitet wurde. Danach saugte man vom ausgefallenen Feststoff ab, wusch dreimal mit Wasser, trocknete und engte ein. Das erhaltene Öl wurde mit Diethylether versetzt, angerieben und die resultierenden Kristalle abgenutscht. Nach Umlösen aus Isopropanol fielen 10,0 g Kristalle vom Schmp. 146 - 148°C an.

Analog hierzu wurden folgende Verbindungen der Formel I hergestellt:

| Bsp.Nr. | R | Position der CO-Gruppe am Isoxazol-Ring | phys. Daten |
|---|---|---|---|
| 2 | H | 3 | Schmp. 155-157°C |
| 3 | H | 4 | $n_D^{20}$ = 1,546 |
| 4 | 3-Me | 5 | Schmp. 158°C (Zersetzung) |
| 5 | 3-Et | 5 | Kp = 168-173°C/ 0,01 mbar |
| 6 | 5-$CH_2Cl$ | 3 | Schmp. 138 - 143°C |

BAD ORIGINAL

Die neuen Wirkstoffe zeigen eine starke fungitoxische Wirsamkeit gegen phytopathogene Pilze. Sie sind beispielsweise geeignet zur Bekämpfung von Erysiphe graminis (echter Mehltau) in Getreide, Erysiphe cichoracearum (echter Mehltau) an Kürbisgewächsen, Erysiphe polygoni an Bohnen, Podosphaera leucotricha und Phytophthora cactorum an Äpfeln, Phytophthora infestans an Tomaten und Kartoffeln, Phytophthora parasitica an Erdbeeren, Pseudoperonospora cubensis an Gurken, Pseudoperonospora humuli an Hopfen, Peronospora destructor an Zwiebeln, Peronospora tabacina an Tabak, Peronospora sparsa an Rosen, Plasmopara viticola an Reben, Plasmopara halstedii an Sonnenblumen, Sclerospora macrospora an Mais, Bremia lactucae an Salat, Mucor mucedo an Früchten, Rhizopus nigricans an Rüben, Uncinula necator an Reben und Sphaerotheca pannosa an Rosen.

Die neuen Wirkstoffe schädigen Kulturpflanzen in den zur Bekämpfung von Pilzen und Bakterien notwendigen Konzentrationen nicht. Aus diesen Gründen sind sie für den Gebrauch als Pflanzenschutzmittel zur Bekämpfung von Pilzen geeignet.

Die Aufwandmengen liegen je nach Art des gewünschten Effektes zwischen 0,1 und 5 kg Wirkstoff je ha. Ein Teil der Wirkstoffe zeigt kurative Eigenschaften, d.h. die Anwendung der Mittel kann noch nach erfolgter Infektion der Pflanzen durch die Krankheitserreger vorgenommen werden, um einen sicheren Bekämpfungserfolg zu erzielen. Darüber hinaus sind viele der neuen Verbindungen systemisch wirksam, so daß über die Wurzelbehandlung auch ein Schutz oberirdischer Pflanzen möglich ist.

Ferner lassen sich mit den neuen Verbindungen auch Pilze, die Keimlings- und Auflaufkrankheiten hervorrufen, beispielsweise Pythium- und Aphanomyces-Arten an Leguminosen

BAD ORIGINAL

und Baumwolle, bekämpfen. Die Aufwandmengen betragen je 100 kg Saatgut 10 bis 200 g Wirkstoff; die Anwendung erfolgt in Form von Saatgutbeizmitteln.

Die Verbindungen werden angewendet, indem man die Pflanzen mit den Wirkstoffen besprüht oder bestäubt oder die Samen der Pflanzen mit den Wirkstoffen behandelt. Die Anwendung erfolgt vor oder nach der Infektion der Pflanzen oder Samen durch die Pilze.

Die erfindungsgemäßen Substanzen können in die üblichen Formulierungen wie Lösungen, Emulsionen, Suspensionen, Stäube, Pulver, Pasten und Granulate übergeführt werden. Die Anwendungsformen richten sich ganz nach den Verwendungszwecken; sie sollten in jedem Falle eine feine und gleichmäßige Verteilung der wirksamen Substanzen gewährleisten. Die Formulierungen werden in bekannter Weise hergestellt, z.B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel als Hilfslösungsmittel verwendet werden können. Als Hilfsstoffe kommen dafür im wesentlichen Lösungsmittel wie Aromaten (z.B. Xylol, Benzol), chlorierte Aromaten (z.B. Chlorbenzole), Paraffine (z.B. Erdölfraktionen), Alkohole (z.B. Methanol, Butanol), Amine (z.B. Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel wie nichtionogene und anionische Emulgatoren (z.B. Polyoxyethylen - Fettalkohol - Ether, Alkylsulfonate und Arylsulfonate) und Dispergiermittel, wie Lignin, Sulfitablaugen und Methylcellulose in Betracht.

BAD ORIGINAL

Die fungiziden Mittel enthalten 0,1 bis 95 Gew.% Wirkstoff, vorzugsweise 0,5 bis 90 Gew.%.

Die Mittel bzw. die daraus hergestellten gebrauchsfertigen Zubereitungen, wie Lösungen, Emulsionen, Suspensionen, Pulver, Stäube, Pasten oder Granulate werden in bekannter Weise angewendet, beispielsweise durch Versprühen, Vernebeln, Verstäuben, Verstreuen, Beizen oder Gießen.

Beispiele für solche Zubereitungen sind:

I. Man vermischt 90 Gew.-Teile des Wirkstoffs Nr. 1 mit 10 Gew.-Teilen N-Methyl-alpha-pyrrolidon und erhält eine Lösung, die zur Anwendung in Form kleinster Tropfen geeignet ist.

II. 20 Gew.-Teile der Verbindung des Wirkstoffs Nr. 1 werden in einer Mischung gelöst, die aus 80 Gew.-Teilen Xylol, 10 Gew.-Teilen des Anlagerungsproduktes von 8 bis 10 Mol Ethylenoxid an 1 Mol Ölsäure-N-monoethanolamin, 5 Gew.-Teilen Calciumsalz der Dodecylbenzolsulfonsäure und 5 Gew.-Teilen des Anlagerungsproduktes von 40 Mol Ethylenoxid an 1 Mol Ricinusöl besteht. Durch Ausgießen und feines Verteilen der Lösung in 100 000 Gew.-Teilen Wasser erhält man eine wäßrige Dispersion, die 0,02 Gew.% des Wirkstoffs enthält.

III. 20 Gew.-Teile des Wirkstoffs Nr. 4 werden in einer Mischung gelöst, die aus 40 Gew.-Teilen Cyclohexanon, 30 Gew.-Teilen Isobutanol, 20 Gew.-Teilen des Anlagerungsproduktes con 40 Mol Ethylenoxid an 1 Mol Ricinusöl besteht. Durch Eingießen und feines Verteilen der Lösung in 100 000 Gew.-Teilen Wasser erhält man eine wäßrige Dispersion, die 0,02 Gew.% des Wirkstoffs enthält.

BAD ORIGINAL

IV. 20 Gew.-Teile des Wirkstoffs Nr. 2 werden in einer Mischung gelöst, die aus 25 Gew.-Teilen Cyclohexanol, 65 Gew.-Teilen einer Mineralölfraktion vom Siedepunkt 210 bis 280°C und 10 Gew.-Teilen des Anlagerungsproduktes von 40 Mol Ethylenoxid an 1 Mol Ricinusöl besteht. Durch Eingießen und feines Verteilen der Lösung in 100 000 Gew.-Teilen Wasser erhält man eine wäßrige Dispersion, die 0,02 Gew.% des Wirkstoffs enthält.

V. 20 Gew.-Teile des Wirkstoffs Nr. 5 werden mit 3 Gew.-Teilen des Natriumsalzes der Diisobutylnaphthalin-alpha-sulfonsäure, 17 Gew.-Teilen des Natriumsalzes einer Ligninsulfonsäure aus einer Sulfitablauge und 60 Gew.-Teilen pulverförmigem Kieselsäuregel gut vermischt und in einer Hammermühle vermahlen. Durch feines Verteilen der Mischung in 20 000 Gew.-Teilen Wasser erhält man eine Spritzbrühe, die 0,1 Gew.% des Wirkstoffs enthält.

VI. 3 Gew.-Teile des Wirkstoffs Nr. 3 werden mit 97 Gew.-Teilen feinteiligem Kaolin innig vermischt. Man erhält auf diese Weise ein Stäubemittel, das 3 Gew.% des Wirkstoffs enthält.

VII. 30 Gew.-Teile des Wirkstoffs Nr. 4 werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhält auf diese Weise eine Aufarbeitung des Wirkstoffs mit guter Haftfähigkeit.

VIII. 40 Gew.-Teile des Wirkstoffs Nr. 2 werden mit 10 Teilen Natriumsalz eines Phenolsulfonsäure-harnstoff-formaldehyd-Konensates, 2 Teilen Kieselgel und 48 Teilen Wasser innig vermischt. Man erhält eine sta-

BAD ORIGINAL

bile wäßrige Dispersion. Durch Verdünnen mit 100 000 Gew.-Teilen Wasser erhält man eine wäßrige Dispersion, die 0,04 Gew.% Wirkstoff enthält.

IX. 20 Teile des Wirkstoffs Nr. 4 werden mit 2 Teilen Calciumsalz der Dodecylbenzolsulfonsäure, 8 Teilen Fettalkohol-polyglykolether, 2 Teilen Natriumsalz eines Phenolsulfonsäure-harnstoff-formaldehyd-Kondensats und 68 Teilen eines paraffinischen Mineralöls innig vermischt. Man erhält eine stabile ölige Dispersion.

Die erfindungsgemäßen Mittel können in diesen Anwendungsformen auch zusammen mit anderen Wirkstoffen vorliegen, wie z.B. Herbiziden, Insektiziden, Wachstumsregulatoren und anderen Fungiziden, oder auch mit Düngemitteln vermischt und ausgebracht werden. Beim Vermischen mit Fungiziden erhält man dabei in vielen Fällen eine Vergrößerung des fungiziden Wirkungsspektrums.

Die folgende Liste von Fungiziden, mit denen die erfindungsgemäßen Verbindungen kombiniert werden können, soll die Kombinationsmöglichkeiten erläutern, nicht aber einschränken.

Fungizide, die mit den erfindungsgemäßen Verbindungen kombiniert werden können, sind beispielsweise:

Dithiocarbamate und deren Derivate, wie
Ferridimethyldithiocarbamat,
Zinkdimethyldithiocarbamat,
Manganethylenbisdithiocarbamat,
Mangan-Zink-ethylendiamin-bis-dithiocarbamat,
Zinkethylenbisdithiocarbamat,
Tetramethylthiuramdisulfide,
Ammoniak-Komplex von Zink-(N,N-ethylen-bis-dithiocarbamat)

BAD ORIGINAL

und
N,N'-Polyethylen-bis-(thiocarbamoyl)-disulfid,
Zink-(N,N'-propylen-bis-dithiocarbamat),
Ammoniak-Komplex von Zink-(N,N'-propylen-bis-dithiocarbamat)
und
N,N'-Polypropylen-bis-(thiocarbamoyl)-disulfid;

Nitroderivate, wie
Dinitro-(1-methylheptyl)-phenylcrotonat,
2-sec-Butyl-4,6-dinitrophenyl-3,3-dimethylacrylat,
2-sec-Butyl-4,6-dinitrophenyl-isopropylcarbonat;

heterocyclische Strukturen, wie
N-Trichlormethylthio-tetrahydrophthalamid,
N-Trichlormethylthio-phthalimid,
2-Heptadecyl-2-imidazolin-acetat,
2,4-Dichlor-6-(o-chloranilino)-s-triazin,
O,O-Diethyl-phthalimidophosphonothionat,
5-Amino-1-(bis-(dimethylamino)-phosphinyl)-3-phenyl-1,2,4-
-triazol,
5-Ethoxy-3-trichlormethyl-1,2,4-thiadiazol,
2,3-Dicyano-1,4-dithioanthrachinon,
2-Thio-1,3-dithio-(4,5-b)-chinoxalin,
1-(Butylcarbamoyl)-2-benzimidazol-carbaminsäuremethylester,
2-Methoxycarbonylamino-benzimidazol,
2-Rhodanmethylthio-benzthiazol,
4-(2-Chlorphenylhydrazono)-3-methyl-5-isoxazolon,
Pyridin-2-thio-1-oxid,
8-Hydroxychinolin bzw. dessen Kupfersalz,
2,3-Dihydro-5-carboxanilido-6-methyl-1,4-oxathiin-4,4-dioxid,
2,3-Dihydro-5-carboxanilido-6-methyl-1,4-oxathiin,
2-(Furyl-(2))-benzimidazol,
Piperazin-1,4-diyl-bis-(1-(2,2,2-trichlor-ethyl)-formamid),
2-(Thiazolyl-(4))-benzimidazol,

5-Butyl-2-dimethylamino-4-hydroxy-6-methyl-pyrimidin,
Bis-(p-Chlorphenyl)-3-pyridinmethanol,
1,2-Bis-(3-ethoxycarbonyl-2-thioureido)-benzol,
1,2-Bis-(3-methoxycarbonyl-2-thioureido)-benzol

und verschiedene Fungizide, wie
Dodecylguanidinacetat,
3-(3-(3,5-Dimethyl-2-oxycyclohexyl)-2-hydroxyethyl)-glutarimid,
Hexachlorbenzol,
N-Dichlorfluormethylthio-N',N'-dimethyl-N-phenyl-schwefelsäureamid,
2,5-Dimethyl-furan-3-carbonsäureanilid,
2,5-Dimethyl-furan-3-carbonsäure-cyclohexylamid,
2-Methyl-benzoesäure-anilid,
2-Iod-benzoesäure-anilid,
1-(3,4-Dichloranilino)-1-formylamino-2,2,2-trichlorethan,
2,6-Dimethyl-N-tridecyl-morpholin bzw. dessen Salze,
2,6-Dimethyl-N-cyclododecyl-morpholin bzw. dessen Salze.

5-Nitro-isophthalsäure-di-isopropylester,
1-(1',2',4'-Triazolyl-1')-[1-(4'-chlorphenoxy)]-3,3-dimethylbutan-2-on,
1-(1',2',4'-Triazolyl-1')-[1-(4'-chlorphenoxy)]-3,3-dimethylbutan-2-ol,
N-(n-Propyl)-N-(2,4,6-trichlorphenoxyethyl)-N'-imidazolyl-harnstoff,
N-Cyclohexyl-N-methoxy-2,5-dimethyl-furan-3-carbonsäureamid,
2,4,5-Trimethyl-furan-3-carbonsäureanilid,
5-Methyl-5-vinyl-3-(3,5-dichlorphenyl)-2,4-dioxo-1,3-oxazolidin,
5-Methoxymethyl-5-methyl-3-(3,5-dichlorphenyl)-2,4-dioxo-1,3-ixazolidin,

N-[3-(p-tert.-Butylphenyl)-2-methyl-propyl]-cis-2,6-dimethylmorpholin.

Die folgenden Beispiele belegen die biologische Wirkung der neuen Verbindungen der Formel I. Als Vergleichsmittel dienten die fungiziden Wirkstoffe N-(1'-Methoxycarbonyl-ethyl)-N-(furan-(2")-carbonyl)-2,6-dimethylanilin (A; DE-OS 25 13 788) und N-(1'-Methoxycarbonylethyl)-N-(pyridin-3"-carbonyl)-2-methyl-6-chloranilin (B; DE-OS 25 13 732).

Beispiel A

Wirksamkeit gegen Phytophthora infestans an Tomaten

Blätter von Topfpflanzen der Sorte "Große Fleischtomate" werden mit wäßriger Spritzbrühe, die 80 % Wirkstoff und 20 % Emulgiermittel (Natriumligninsulfonat) in der Trockensubstanz enthält, besprüht. Nach dem Antrocknen des Spritzbelages werden die Blätter mit einer Zoosporenaufschwemmung des Pilzes Phytophthora infestans infiziert. Die Pflanzen werden dann in einer wasserdampfgesättigten Kammer bei Temperaturen zwischen 16 und 18°C aufgestellt. Nach 5 Tagen hat sich die Krankheit auf den unbehandelten, jedoch infizierten Kontrollpflanzen so stark entwickelt, daß die fungizide Wirksamkeit der Substanzen beurteilt werden kann.

In diesem Versuch zeigten beispielsweise die Substanzen der Beispiele 1 und 2 bei der Anwendung als 0,025%ige oder 0,006%ige Spritzbrühe eine bessere fungizide Wirkung (beispielsweise 97 %) als die Vergleichssubstanzen A und B (beispielsweise 50 %).

Beispiel B

Wirksamkeit gegen Plasmopara viticola

Blätter von Topfreben der Sorte "Müller-Thurgau" werden mit wäßriger Spritzbrühe, die 80 % Wirkstoff und 20 % Emulgiermittel (Natriumligninsulfonat) in der Trockensubstanz enthält, besprüht. Um die Wirkungsdauer der Wirkstoffe beurteilen zu können, werden die Pflanzen nach dem Antrocknen des Spritzbelages 10 Tage im Gewächshaus aufgestellt. Erst dann werden die Blätter mit einer Zoosporenaufschwemmung von Plasmopara viticola (Rebenperonospora) infiziert. Danach werden die Reben zunächst für 16 h in einer wasserdampfgesättigten Kammer bei 24°C und anschließend für 8 Tage in einem Gewächshaus mit Temperaturen zwischen 20 und 30°C aufgestellt. Nach dieser Zeit werden die Pflanzen zur Beschleunigung des Sporangienträgerausbruches abermals für 16 h in der feuchten Kammer aufgestellt. Dann erfolgt die Beurteilung des Ausmaßes des Pilzausbruches auf den Blattunterseiten.

In diesem Versuch zeigten beispielsweise die Substanzen der Beispiele 1 und 3 bei der Anwendung als 0,025%ige Spritzbrühe eine sehr gute fungizide Wirkung (beispielsweise 100 %).

Patentansprüche

1. Isoxazolyl-carbonsäure-amid der Formel I

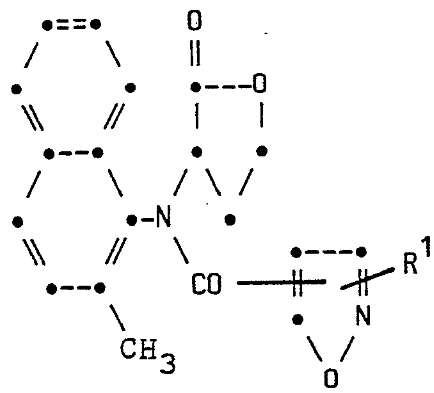

I,

in der $R^1$ für Wasserstoff, Methyl, Ethyl oder Chlormethyl steht.

2. N-(alpha-Butyro-gamma-lactonyl)-N-(2-methyl-1-naphthyl)-isoxazol-5-carbonsäureamid.

3. N-(alpha-Butyro-gamma-lactonyl)-N-(2-methyl-1-naphthyl)-isoxazol-3-carbonsäureamid.

4. Verfahren zur Herstellung der Isoxazolyl-carbonsäureamide der Formel I gemäß Anspruch 1, dadurch gekennzeichnet, daß man N-(alpha-Butyro-gamma-lactonyl)-2-methyl-1-naphthylamin mit einem Isoxazol-carbonsäurederivat der Formel II

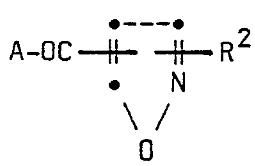

II,

in der $R^2$ für Wasserstoff, Methyl, Ethyl oder Chlormethyl und A für eine nucleophil verdrängbare Abgangsgruppe steht, umsetzt.

5. Fungizid, enthaltend eine Verbindung der Formel I gemäß Anspruch 1.

6. Fungizid, enthaltend eine Verbindung der Formel I gemäß Anspruch 1 und einen festen oder flüssigen Trägerstoff.

7. Verfahren zur Bekämpfung von Pilzen, dadurch gekennzeichnet, daß man eine fungizid wirksame Menge einer Verbindung der Formel I gemäß Anspruch 1 auf durch Pilzbefall bedrohte Flächen, Pflanzen und Saatgüter oder auf phytopathogene Pilzen einwirken läßt.

Patentansprüche (für Österreich)

1. Fungizid, enthaltend ein Isoxazolyl-carbonsäure-amid der Formel I

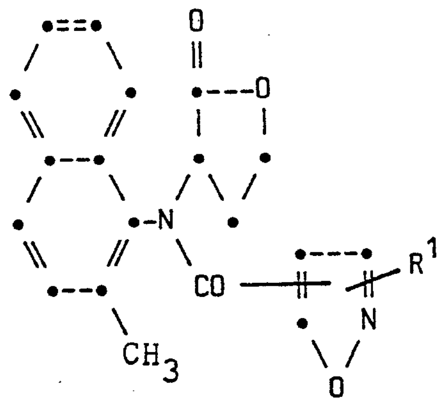

I,

in der $R^1$ für Wasserstoff, Methyl, Ethyl oder Chlormethyl steht.

2. Fungizid, enthaltend N-(alpha-Butyro-gamma-lactonyl)-N-(2-methyl-1-naphthyl)-isoxazol-5-carbonsäureamid.

3. Fungizid, enthaltend N-(alpha-Butyro-gamma-lactonyl)-N-(2-methyl-1-naphthyl)-isoxazol-3-carbonsäureamid.

4. Verfahren zur Herstellung der Isoxazolyl-carbonsäureamide der Formel I gemäß Anspruch 1, dadurch gekennzeichnet, daß man N-(alpha-Butyro-gamma-lactonyl)-2-methyl-1-naphthylamin mit einem Isoxazol-carbonsäurederivat der Formel II

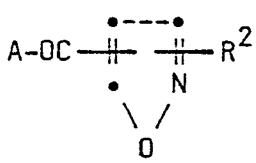

II,

in der $R^2$ für Wasserstoff, Methyl, Ethyl oder Chlormethyl und A für eine nucleophil verdrängbare Abgangsgruppe steht, umsetzt.

5. Fungizid, enthaltend eine Verbindung der Formel I gemäß Anspruch 1 und einen festen oder flüssigen Trägerstoff.

6. Verfahren zur Bekämpfung von Pilzen, dadurch gekennzeichnet, daß man eine fungizid wirksame Menge einer Verbindung der Formel I gemäß Anspruch 1 auf durch Pilzbefall bedrohte Flächen, Pflanzen und Saatgüter oder auf phytopathogene Pilzen einwirken läßt.

Europäisches Patentamt

**EUROPÄISCHER RECHERCHENBERICHT**

0080080 Nummer der Anmeldung

EP 82 11 0076

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| | --- | | C 07 D 413/12<br>A 01 N 43/80 |
| Y | EP-A-0 029 996 (BASF)<br>* Ansprüche * | 1,4-7 | |
| | --- | | |
| Y | EP-A-0 037 927 (BASF)<br>* Ansprüche * | 1,4-7 | |
| | --- | | |
| Y | FR-A-2 407 210 (CHEVRON RESEARCH COMPANY)<br>* Ansprüche 1,6-8 * | 1,5-7 | |
| | --- | | |
| Y | EP-A-0 018 510 (CIBA-GEIGY)<br>* Ansprüche 1,11-13 * | 1,5-7 | |
| | ----- | | |
| | | | RECHERCHIERTE SACHGEBIETE (Int. Cl. 3) |
| | | | C 07 D 413/00<br>C 07 D 261/00 |

Der vorliegende Recherchenbericht wurde für alle Patentansprüche erstellt.

| Recherchenort | Abschlußdatum der Recherche | Prüfer |
|---|---|---|
| DEN HAAG | 03-02-1982 | HENRY J.C. |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503. 03.82